# FASCICULE DE BREVET EUROPEEN

(11) **EP 3 347 339 B1**
(45) Date de publication et mention de la délivrance du brevet: **24.04.2024**
(21) Numéro de dépôt: 16775296.3
(22) Date de dépôt: 09.09.2016
(51) Int. Cl.: C07C 213/02, C07C 217/08, C07C 255/13, C07C 255/25, C02F 5/12, C10M 133/06, C08L 63/00, B03D 1/01, B03D 1/004, C08G 65/26, C08G 65/333

(54) **COMPOSÉS ÉTHERAMINES ET LEUR UTILISATION EN TANT QUE COLLECTEUR DE FLOTTATION**
ETHERAMINVERBINDUNGEN UND DEREN VERWENDUNG ALS FLOTATIONSSAMMLER
ETHER AMINE COMPOUNDS AND USE THEREOF AS FLOTATION COLLECTOR

(30) Priorité: 10.09.2015 FR 1558435
(43) Date de publication de la demande: 18.07.2018
(73) Titulaire: Arkema France, 92700 Colombes (FR)
(72) Inventeur: GILLET, Jean-Philippe, 69530 Brignais (FR); JORDA, Eric, 69005 Lyon (FR)
(86) Numéro de dépôt international: PCT/FR2016/052277
(87) Numéro de publication internationale: WO 2017/042514

(56) Documents cités:
- EP-A1- 0 533 552
- EP-A1- 0 568 156
- EP-A1- 1 219 597
- WO-A1-2005/014491
- US-A- 4 324 739
- US-B1- 6 260 561
- WATANABE S ET AL: "CHARACTERISTIC PROPERTIES OF CUTTING FLUID ADDITIVES DERIVED FROM FATTY ALCOHOLS", JOURNAL OF THE AMERICAN OIL CHEMISTS' SOCIETY (JAOCS), SPRINGER, DE, vol. 68, no. 1, 1 janvier 1991 (1991-01-01), pages 44-46, XP001207326, ISSN: 0003-021X, DOI: 10.1007/BF02660308
- SÉBASTIEN PERDRIAU ET AL: "A Metal-Ligand Cooperative Pathway for Intermolecular Oxa-Michael Additions to Unsaturated Nitriles", ANGEWANDTE CHEMIE INTERNATIONAL EDITION, vol. 54, no. 14, 27 mars 2015 (2015-03-27) , pages 4236-4240, XP055270087, DE ISSN: 1433-7851, DOI: 10.1002/anie.201412110
- "Alkoxypropylamines prepn - by hydrogenation of crude alkoxypropionitrile on Raney nickel at 110-140 deg. C", DERWENT, 1974, XP002192710,
- T. S. PATTERSON: "LIII.-The influence of solvents on the rotation of optically active compounds. Part II. Influence of isobutyl alcohol and of sec.octyl alcohol (methylhexylcarbinol) on ethyl tartrate", JOURNAL OF THE CHEMICAL SOCIETY, TRANSACTIONS, vol. 79, no. 0, 1 January 1901 (1901-01-01), pages 477-493, XP055740107, GB ISSN: 0368-1645, DOI: 10.1039/CT9017900477
- Anonymous: "CAS No.64774-45-4,2(5H)-Furanone, 3,4-dichloro-5-(sec-octyloxy)- Suppliers", LookChem, 1 January 2008 (2008-01-01), pages 1-2, XP055790247, Retrieved from the Internet: URL:https://www.lookchem.com/cas-647/64774 -45-4.html [retrieved on 2021-03-26]
- Watanabe S. ET AL: "Characteristic properties of cutting fluid additives derived from fatty alcohols", Journal of the American Oil Chemists' Society, vol. 68, no. 1, 1 January 1991 (1991-01-01), pages 44-46, XP055790243, DE ISSN: 0003-021X, DOI: 10.1007/BF02660308 Retrieved from the Internet: URL:https://api.wiley.com/onlinelibrary/td m/v1/articles/10.1007%2FBF02660308>
- Jeffrey H: "Colorant compounds", US7381831 B1, 1 January 2008 (2008-01-01), pages 1-2, XP055790241,
- Westland: "N-substituted derivatives of 2-aminoethanethiol and 2-hydrazinoethanethiol", Journal of medicinal chemistry, vol. 11, no. 4, 1 January 1968 (1968-01-01), pages 824-829, XP055790242,
- "what-is-the-logical-way-to-understand-iso -sec-tert-and-neo-prefixes", , Retrieved from the Internet: URL:https://chemistry.stackexchange.com/qu estions/111078/what-is-the-logical-way-to- understand-iso-sec-tert-and-neo-prefixes [retrieved on 2021-02-21]
- "common_name", , Retrieved from the Internet: URL:ttp://www.chem.ucla.edu/~harding/IGOC/ C/common_name.html [retrieved on 2021-02-21]

## Description

La présente invention concerne le domaine général des étheramines. La famille des étheramines représente une famille unique de produits chimiques offrant un large éventail de propriétés.

En effet, elles peuvent être utilisées notamment en tant que lubrifiant, tensioactif cationique, collecteur de flottation pour les minerais ou encore en tant qu'inhibiteur de corrosion. Ainsi, elles constituent une classe de matériaux revêtant un intérêt industriel majeur pour de nombreux acteurs. Le marché des étheramines est d'ailleurs déjà largement développé depuis plusieurs décennies.

Les étheramines sont classiquement synthétisées en faisant réagir tout d'abord un alcool avec un composé nitrile, généralement l'acrylonitrile, en présence d'un catalyseur basique. Une étape d'hydrogénation du produit obtenu est ensuite réalisée afin d'isoler l'étheramine visée.

Ainsi, le brevet US5196589 décrit un procédé de fabrication d'une étheramine en faisant réagir tout d'abord un alcool avec l'acrylonitrile, en présence d'un catalyseur alcalin. Une étape d'hydrogénation est ensuite réalisée afin d'obtenir l'étheramine désirée. La particularité de ce procédé réside dans le fait que l'alcool, un composé comprenant un nombre d'atomes de carbone allant de 6 à 36, est en présence d'un composé radical libre stable, permettant ainsi de notamment réduire considérablement l'obtention de produits secondaires non désirés.

Les travaux de S Watanabe et coll. (JAOCS, Vol. 68, no. 1 (1991), 44-46) décrivent des 3-aminopropyl octyl-, decyl- et dodecyl-éthers présentant de bonnes propriétés lubrifiantes et anti-microbiennes et qui peuvent être utilisés en tant qu'huiles de coupe à base aqueuse.

S. Perdriau et coll. (Angew. Chem. Int. Ed. (2015), 54, 4236-4240) présentent une nouvelle voie catalytique pour des réactions d'addition oxaMichael d'alcools en nitriles insaturés utilisant un catalyseur spécifique au ruthénium.

Le document JP48103505 (1973) divulgue quant à lui que de l'acrylonitrile et des alcools mono- ou dihydriques sont traités, en présence d'hydroxydes alcalins pour donner des alkoxypropionitriles brutes, qui sont lavées à l'eau afin de retirer l'excès d'hydroxydes, avant d'hydrogéner le produit brut avec du nickel ou du coblat de Raney pour conduire aux alkoxypropylamines.

Le brevet EP1219597 divulgue quant à lui un procédé de préparation d'une étheramine comprenant une première étape de réaction d'un alcool primaire ou secondaire avec l'acrylonitrile, en présence d'un hydroxyde de métal alcalin, puis une seconde étape d'hydrogénation du produit obtenu. L'alcool primaire ou secondaire est un composé comprenant un nombre d'atomes de carbone allant de 6 à 24.

En revanche, à l'heure où l'enjeu environnemental est véritablement important, aucun de ces documents n'indique utiliser un quelconque réactif bio-sourcé ou biodégradable et présentant un bon profil écotoxicologique.

En outre, il est connu que des étheramines particulières, notamment les produits commercialisés Tomamine^{®} PA-14 et Tomamine^{®} DA-14, sont utilisées en vue de l'élimination sélective de silicates lors de la flottation de minerais.

Il serait donc intéressant de fournir une étheramine obtenue à partir d'au moins un réactif bio-sourcé et biodégradable. Il serait également avantageux que l'utilisation d'une telle étheramine conduise à une élimination plus sélective de silicates lors de la flottation de minerais que les étheramines commerciales.

La présente invention a pour objet un composé de formule (I) : 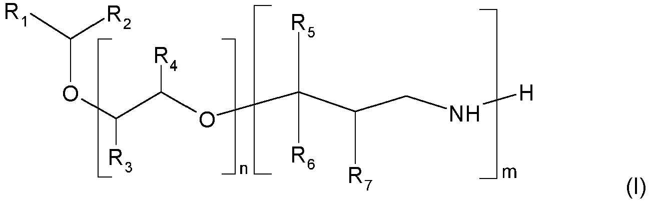 dans laquelle :
- R₁ est un groupement hexyle, R₂ est un groupement méthyle,
- les groupements R₃ et R₄, identiques ou différents, sont choisis, indépendamment l'un de l'autre, parmi l'atome d'hydrogène, le groupement méthyle et le groupement éthyle ;
- les groupements R₅, R₆ et R₇ représentent chacun l'atome d'hydrogène ;
- n est 0 ;
- m est un nombre entier allant de 1 à 6, et m est différent de 1.

Dans un autre mode de réalisation, l'invention a pour objet un composé de formule (I) : 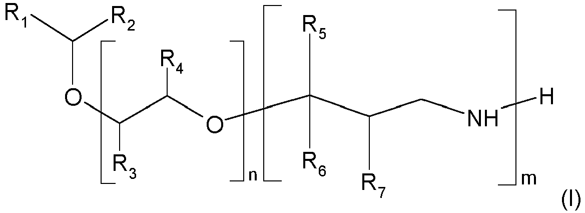 dans laquelle :
R₁ et R₂, identiques ou différents, représentent, indépendamment l'un de l'autre, un groupement hydrocarboné, linéaire, ramifié ou cyclique, saturé ou insaturé, comprenant de 1 à 15 atomes de carbone, de préférence de 1 à 10 atomes de carbone ;
R₃ et R₄, identiques ou différents, sont choisis, indépendamment l'un de l'autre, parmi l'atome d'hydrogène, le groupement méthyle et le groupement éthyle ;
R₅, R₆, et R₇, identiques ou différents, sont choisis, indépendamment l'un de l'autre,
parmi l'atome d'hydrogène et un groupement alkyle comprenant de 1 à 6 atomes de carbone, de préférence de 1 à 4 atomes de carbone, de préférence encore de 1 à 3 atomes de carbone ;
n représente 1 ;
m est un nombre entier allant de 1 à 6.

Selon un mode de réalisation préféré, le composé de formule (I) ci-dessus est caractérisé en ce que les groupements R₃ et R₄, identiques ou différents, sont choisis, indépendamment l'un de l'autre, parmi l'atome d'hydrogène et le groupement méthyle.

La présente invention a également pour objet un procédé de fabrication du composé de formule (I) selon l'invention.

Un autre objet de l'invention est un composé de formule (V) : 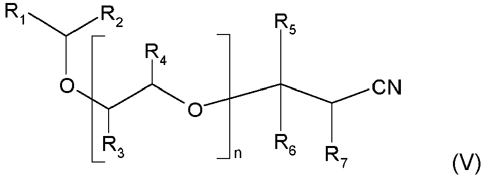 dans laquelle :
- les groupements R₁, R₂, R₃, R₄, R₅, R₆ et R₇ sont tels que définis précédemment, et
- n est tel que défini précédemment.

La présente invention a également pour objet un composé de formule (VI) : 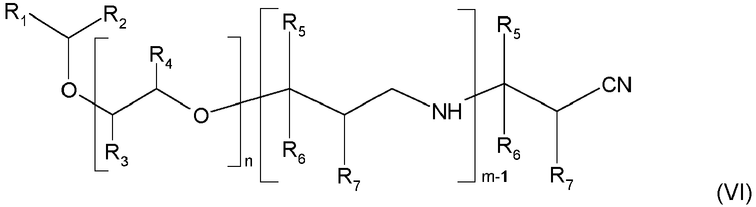 dans laquelle :
- les groupements R₁, R₂, R₃, R₄, R₅, R₆ et R₇ sont tels que définis précédemment, et
- n et m sont tels que définis précédemment.

Un autre objet de la présente invention concerne l'utilisation du composé selon l'invention en tant que notamment collecteur de flottation pour les minerais.

D'autres avantages et caractéristiques de l'invention apparaîtront plus clairement à l'examen de la description détaillée.

Il est par ailleurs précisé que les expressions « compris entre ... et ... » et « de... à... » utilisées dans la présente description doivent s'entendre comme incluant chacune des bornes mentionnées.

L'invention a également pour objet un procédé de fabrication du composé de formule (I) selon l'invention, comprenant successivement :
- une étape de réaction d'un composé de formule (II) : 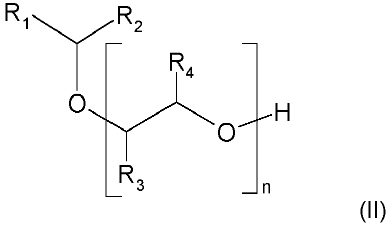 dans laquelle les groupements R₁, R₂, R₃ et R₄ et n sont tels que définis précédemment ; avec un nitrile α,β-insaturé ;
- une réaction d'hydrogénation ;
- le produit issu de ces étapes étant susceptible de réagir en série (m-1) fois avec le nitrile α,β-insaturé, puis du dihydrogène, m étant tel que défini précédemment.

Selon un mode de réalisation préféré, le nitrile α,β-insaturé est choisi parmi l'acrylonitrile et le méthacrylonitrile, de préférence l'acrylonitrile.

Ainsi, lorsque n est égal à 0, le composé de formule (II) peut être le 2-octanol. Cet alcool présente un intérêt particulier à plusieurs titres. En effet, il s'agit d'un produit bio-sourcé, biodégradable et présentant un bon profil écotoxicologique. En outre, le point d'ébullition du 2-octanol est élevé et son prix de revient est tout à fait raisonnable.

Avantageusement, le ratio molaire du nitrile α,β-insaturé sur le composé de formule (II) varie de 0,8 à 1,2, de préférence de 0,9 à 1,2.

De manière particulièrement avantageuse, le ratio molaire du nitrile α,β-insaturé sur le composé de formule (II) varie de 1,01 à 1,1, c'est-à-dire que la réaction du composé de formule (II) avec le nitrile α,β-insaturé est conduite avec un léger excès de nitrile α,β-insaturé.

Préférentiellement, la réaction du composé de formule (II) avec le nitrile α,β-insaturé est réalisée en présence d'au moins un catalyseur basique CB.

De manière préférée, le catalyseur basique CB est choisi parmi les hydroxydes d'alcalins et d'alcalino-terreux, les alcoolates alcalins, les hydrures d'alcalins, les résines basiques et les hydroxydes d'ammonium quaternaires.

De manière particulièrement préférée, le catalyseur basique CB est choisi parmi l'hydroxyde de sodium, l'hydroxyde de potassium, l'hydrure de sodium et l'hydrure de potassium.

Selon un mode de réalisation particulier de l'invention, la quantité de catalyseur basique CB utilisée varie de 0,1% à 2% en poids, de préférence de 0,5% à 1% en poids, par rapport au poids total du composé de formule (II).

La température de la réaction du composé de formule (II) avec le nitrile α,β-insaturé peut varier dans de grandes proportions. De préférence, elle varie de 20 à 75°C, plus préférentiellement de 25 à 70°C, encore plus préférentiellement de 25 à 65°C.

Avantageusement, la réaction du composé de formule (II) avec le nitrile α,β-insaturé est réalisée sans solvant mais il est également possible d'utiliser un solvant neutre vis-à-vis de la réaction du composé de formule (II) avec le nitrile α,β-insaturé.

On entend par « solvant neutre vis-à-vis de la réaction » tout solvant qui n'interagit pas chimiquement avec les réactifs de la réaction du composé de formule (II) avec le nitrile α,β-insaturé.

De manière préférée, les solvants neutres vis-à-vis de la réaction sont choisis parmi les éthers, le diméthylformamide et les solvants aromatiques solubilisant les réactifs choisis parmi le toluène et les xylènes.

Le cas échéant, le catalyseur basique CB peut être neutralisé en fin de réaction par tout moyen connu de l'homme du métier, tel que par exemple, et de manière non limitative, un acide organique ou minéral, de préférence choisi parmi l'acide chlorhydrique et l'acide acétique ou, alternativement, le catalyseur peut être éliminé, par exemple par filtration lorsqu'il est de nature solide.

De manière préférée, la réaction d'hydrogénation est réalisée en présence d'au moins un catalyseur CT.

Selon un mode de réalisation particulier, ledit catalyseur CT est choisi parmi le nickel de Raney et le cobalt de Raney.

Avantageusement, la quantité de catalyseur CT varie de 0,5 à 10% en poids, de préférence de 2 à 8% en poids, par rapport au poids du produit issu de la réaction du composé de formule (II) avec le nitrile α,β-insaturé.

Préférentiellement, la pression lors de la réaction d'hydrogénation varie de 1 à 10 MPa, de préférence de 1,5 à 5 MPa.

De manière préférée, la température de la réaction d'hydrogénation varie de 50 à 170°C, de préférence de 70 à 150°C.

Selon un mode de réalisation particulier de l'invention, afin de favoriser la formation de l'amine primaire, il est envisageable d'adjoindre une quantité d'ammoniac susceptible de générer une pression partielle d'ammoniac. Avantageusement, un ratio molaire ammoniac/fonction nitrile variant de 0,5 à 2 est adapté. De manière alternative, il est également possible d'ajouter une base forte, de préférence choisie parmi l'hydroxyde de sodium et l'hydroxyde de potassium, selon une quantité pouvant varier de 100 ppm à 5000 ppm, de préférence de 500 à 5000 ppm, plus préférentiellement de 500 à 2500 ppm, par rapport à la quantité de produit issu de la réaction du composé de formule (II) avec le nitrile α,β-insaturé.

De manière particulièrement avantageuse, l'adjonction de ladite quantité d'ammoniac susceptible de générer une pression partielle d'ammoniac et l'ajout de ladite base forte sont combinés.

De préférence, les étapes successives de réaction du composé de formule (II) avec le nitrile α,β-insaturé et de réaction d'hydrogénation sont réalisées dans le même réacteur.

Il est également possible de travailler en milieu solvant avec des solvants organiques ou hydro-organiques, tels que par exemple les alcools (méthanol, éthanol, isopropanol) et tout autre solvant utilisé pour les réactions d'hydrogénation et solubilisant les réactifs et les produits finaux.

Il est possible de travailler en batch en introduisant la totalité des réactifs et en conduisant la réaction d'hydrogénation. Il est également possible de travailler en semi-batch en chargeant le solvant, l'ammoniac et/ou la base forte, le catalyseur et l'hydrogène, puis en introduisant en continu le produit de condensation issu de la réaction du composé de formule (II) avec le nitrile α,β-insaturé.

Avantageusement, la réaction du composé de formule (II) avec le nitrile α,β-insaturé et la réaction d'hydrogénation sont réalisées dans des réacteurs différents.

Préférentiellement, le procédé selon l'invention comprend, préalablement aux étapes successives de réaction du composé de formule (II) avec un nitrile α,β-insaturé et d'hydrogénation, une étape de réaction d'un alcool de formule (III) :

R₁-CH(OH)-R₂ (III),

où R₁ et R₂ sont tels que définis précédemment,
avec n composé(s) de formule (IV) : 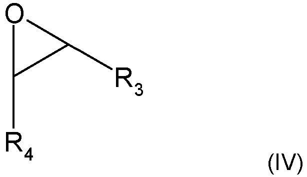 dans laquelle :
   - les groupements R₃ et R₄ sont tels que définis précédemment, et
   - n est tel que défini précédemment.

L'invention a également pour objet l'utilisation d'un composé de formule (I) tel que défini précédemment, en tant que lubrifiant, tensioactif cationique, collecteur de flottation pour les minerais, inhibiteur de corrosion, additif pour carburant et agent de réticulation pour les résines époxy.

L'utilisation du composé de formule (I), en tant que collecteur de flottation pour les minerais est particulièrement préférée.

L'invention est illustrée par les exemples suivants qui ne sont nullement limitatifs.

### EXEMPLES

Le 2-octanol utilisé est le produit de grade Refined commercialisé par Arkema.

### Exemple 1 : Synthèse de la 3-(2-octyloxy)-propanamine

### 1) Synthèse du 3-(2-octyloxy)-propionitrile

### a) Utilisation de l'hydroxyde de potassium

Dans un réacteur équipé d'un agitateur et muni d'une ampoule de coulée, d'un réfrigérant, d'un système d'inertage à l'azote et d'une double enveloppe pour le chauffage, 130 g (1 M) de 2-octanol et 2 g d'hydroxyde de potassium, en solution aqueuse à 50%, sont chargés. Le milieu réactionnel est porté à 45°C sous agitation et sous atmosphère inerte puis 55 g (1,04 M) d'acrylonitrile sont ajoutés goutte à goutte. La température est maintenue après la coulée jusqu'à réaction complète. En fin de réaction, le catalyseur basique est neutralisé par la stoechiométrie en acide chlorhydrique. L'éther propionitrile est isolé par distillation sur film mince avec un rendement molaire de 90%.

### b) Utilisation de l'hydrure de sodium

Dans un réacteur équipé d'un agitateur et muni d'une ampoule de coulée, d'un réfrigérant, d'un système d'inertage à l'azote et d'une double enveloppe pour le chauffage, 130 g (1 M) de 2-octanol et 0,6 g d'hydrure de sodium sont chargés. Le réacteur est purgé à l'azote pour éliminer l'hydrogène formé. Le milieu réactionnel est porté à 35°C sous agitation et sous atmosphère inerte puis 55 g (1,04 M) d'acrylonitrile sont ajoutés goutte à goutte. La température est maintenue après la coulée jusqu'à réaction complète. En fin de réaction, le catalyseur basique est neutralisé par la stoechiométrie en acide chlorhydrique. L'éther propionitrile est isolé par distillation sur film mince avec un rendement molaire de 91%.

### 2) Hydrogénation du 3-(2-octyloxy)-propionitrile

Dans un autoclave de 300 cm³, de type Autoclave Engineer, muni d'un système d'agitation de type turbine auto-aspirante, d'un serpentin de refroidissement et d'un système de régulation de la pression et de la température, 183 g (1 M) de 3-(2-octyloxy)-propionitrile obtenu selon les procédés 1)a) ou 1)b), 14 g de Nickel de Raney et 2000 ppm de KOH, en solution aqueuse à 50%, sont chargés. L'autoclave est verrouillé et purgé à l'azote. Puis, l'hydrogène est introduit lors de la montée en température de telle façon qu'une pression totale de 3 MPa à 110°C soit obtenue. La réaction est poursuivie jusqu'à ce qu'il n'y ait plus de consommation d'hydrogène. En fin de réaction, le catalyseur est récupéré par filtration. Le brut réactionnel est distillé sur film mince pour obtenir la 3-(2-octyloxy)-propanamine avec un rendement molaire de 85%.

### Exemple 2 : Synthèse d'une étherdiamine

### 1) Synthèse du 3-r3-(2-octyloxy)propylaminel-propionitrile

Dans un réacteur équipé d'un agitateur et muni d'une ampoule de coulée, d'un réfrigérant, d'un système d'inertage à l'azote et d'une double enveloppe pour le chauffage, 225 g (1,2 M) de 3-(2-octyloxy)-propanamine et 2,25 g d'eau sont chargés.

Le milieu réactionnel est porté à 60°C sous agitation et sous atmosphère inerte, puis 65 g (1,226 M) d'acrylonitrile sont ajoutés goutte à goutte. La température est maintenue après la coulée jusqu'à réaction complète, soit environ 2 heures. Le 3-[3-(2-octyloxy)propylamine]-propionitrile est obtenu avec un rendement molaire de 87%.

### 2) Hydrogénation du 3-r3-(2-octyloxy)propylaminel-propionitrile

Dans un autoclave de 500 cm³, de type Autoclave Engineer, muni d'un système d'agitation de type turbine auto-aspirante, d'un serpentin de refroidissement et d'un système de régulation de la pression et de la température, 200 g (1 M) de 3-[3-(2-octyloxy)propylamine]-propionitrile obtenu selon le procédé ci-dessus et 3,6 g de Nickel de Raney sont chargés. L'autoclave est verrouillé et purgé à l'azote. Puis, le milieu réactionnel est chauffé jusqu'à 75°C. De l'ammoniac est introduit jusqu'à une pression totale de 0,8 MPa. L'hydrogène est ensuite introduit lors de la montée en température de telle façon qu'une pression totale de 3 MPa à 120°C soit obtenue. La réaction est poursuivie jusqu'à ce qu'il n'y ait plus de consommation d'hydrogène. En fin de réaction, l'autoclave est dégazé et le catalyseur est récupéré par filtration. L'étherdiamine brute est obtenue avec un rendement molaire de 83%.

### Exemple 3 : Synthèse d'une polyétheramine

### 1) Synthèse d'un tris(éther) propionitrile

Dans un réacteur équipé d'un agitateur et muni d'une ampoule de coulée, d'un réfrigérant, d'un système d'inertage à l'azote et d'une double enveloppe pour le chauffage, 262 g (1 M) de 2-octanol tris(éthoxylé) et 2 g d'hydroxyde de potassium, en solution aqueuse à 50%, sont chargés. Le milieu réactionnel est porté à 55°C sous agitation et sous atmosphère inerte, puis 55,6 g (1,05 M) d'acrylonitrile sont ajoutés goutte à goutte. La température est maintenue après la coulée jusqu'à réaction complète. En fin de réaction, le catalyseur basique est neutralisé par la stoechiométrie en acide chlorhydrique. Le tris(éther) propionitrile est obtenu avec un rendement molaire de 89%.

### 2) Hydrogénation du tris(éther) propionitrile

Dans un autoclave de 500 cm³, de type Autoclave Engineer, muni d'un système d'agitation de type turbine auto-aspirante, d'un serpentin de refroidissement et d'un système de régulation de la pression et de la température, 252 g (0,8 M) du tris(éther) propionitrile, obtenu selon le procédé ci-dessus, 20 g de Nickel de Raney et 2000 ppm d'hydroxyde de potassium, en solution aqueuse à 50%, sont chargés. L'autoclave est verrouillé et purgé à l'azote. Puis, l'hydrogène est introduit lors de la montée en température de telle façon qu'une pression totale de 3 MPa à 120°C soit obtenue. La réaction est poursuivie jusqu'à ce qu'il n'y ait plus de consommation d'hydrogène. En fin de réaction, l'autoclave est dégazé et le catalyseur est récupéré par filtration. La tris(éther)amine est obtenue avec un rendement molaire de 81%.

### Exemple 4 : Utilisation de composé selon l'invention en tant que collecteur de flottation

Un minerai de phosphate contenant des silicates est purifié par flottation inverse. Les essais sont réalisés dans une cellule de flottation Outotec.

Dans un premier temps, 2,5 litres d'eau du robinet et 340 g d'un minerai de phosphate broyé (dont la taille des particules varie de 30 à 300 µm) sont introduits. La vitesse de la turbine est ajustée à 1500 tr/min pour assurer une mise en suspension du minerai dans l'ensemble du volume de la cellule. 0,34 g d'acide phosphorique, en solution aqueuse à 85%, sont ensuite ajoutés et une agitation est maintenue pendant trois minutes.

Puis, 0,17 g d'un collecteur de carbonate fourni par la Société CECA sous le nom commercial de Melioran^{®} P312 est ajouté et une agitation est maintenue pendant deux minutes. L'air est ensuite alimenté dans la cellule à un débit de 3 L/min et la flottation est réalisée pendant deux minutes. Une collecte régulière des mousses avec une spatule est réalisée.

L'alimentation d'air est coupée en fin de flottation et 10,2 g de collecteur cationique pour silicates sont ajoutés. Une agitation est maintenue pendant deux minutes avant de rétablir l'alimentation en air. La flottation est réalisée pendant quatre minutes.

À l'issue de ces deux étapes, le minerai restant dans la cellule de flottation est filtré sur Büchner et séché dans une étuve pendant une nuit. Le minerai séché est ensuite pesé pour déterminer la quantité récupérée et envoyé en analyse pour la détermination de sa composition.

Les essais comparatifs portent sur cinq collecteurs cationiques pour les silicates utilisés dans la deuxième étape de flottation.

Le minerai de départ est de type fluoroapatite contenant 43% en poids de calcite et 17% en poids de quartz, par rapport au poids total du minerai, comme impuretés. La teneur en composé P₂O₅ est de 13,8% en poids par rapport au poids du minerai.

Le composé A est un composé comparatif. Il s'agit du Noramac^{®} C26 (acétate de N-alkyl coco amine) commercialisée par la société CECA.

Le composé B est un composé comparatif. Il s'agit de la Tomamine^{®} PA-14 (isodécyloxypropylamine) commercialisée par la société Air Products.

Le composé C est un composé comparatif. Il s'agit de la Tomamine^{®} DA-14 (isodécyloxypropyl-1,3-diaminopropane) commercialisée par la société Air Products.

Le composé D est un composé selon l'invention répondant à la formule (VII) suivante :

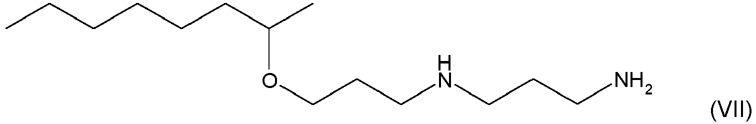

Le composé E est un composé selon l'invention répondant à la formule (VIII) suivante :

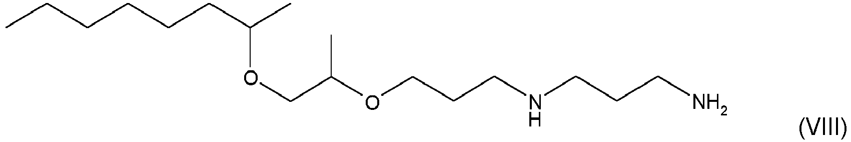

Les résultats d'analyse du minerai après flottation sont résumés dans le Tableau 1 ci-dessous :

**-- Tableau 1 --**

| | Silicates éliminés (% massique) | Calcite éliminée (% massique) | Teneur finale en P₂O₅ (% massique) |
|---|---|---|---|
| Composé A (comp.) | 12,1 | 65,2 | 20,5 |
| Composé B (comp.) | 29,4 | 76,4 | 22,8 |
| Composé C (comp.) | 78,5 | 64,9 | 25,6 |
| **Composé D (inv.)** | 81 | 86,6 | 29,6 |
| **Composé E (inv.)** | 81,6 | 82,9 | 28,6 |

Le tableau 1 montre clairement que les composés D et E selon l'invention permettent d'éliminer une plus grande quantité de silicates que les trois composés comparatifs A, B et C.

En outre, la teneur finale en P₂O₅ est plus importante grâce à l'utilisation des composés D et E que celle associée à l'utilisation des composés comparatifs.

Ainsi, il a été démontré que l'utilisation de composé selon l'invention conduisait à une élimination sélective lors de la flottation d'un minerai de phosphate. Cette propriété est même améliorée par rapport aux produits commerciaux.

## Revendications

1. Composé de formule (I) : 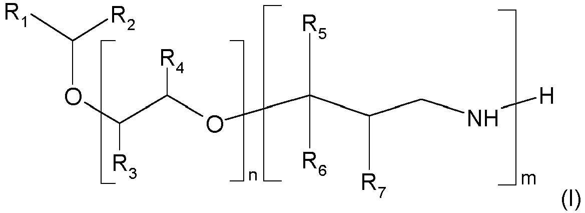 dans laquelle :
- R₁ est un groupement hexyle, R₂ est un groupement méthyle,
- les groupements R₃ et R₄, identiques ou différents, sont choisis, indépendamment l'un de l'autre, parmi l'atome d'hydrogène, le groupement méthyle et le groupement éthyle ;
- les groupements R₅, R₆ et R₇ représentent chacun l'atome d'hydrogène ;
- n est 0 ;
- m est un nombre entier allant de 1 à 6, et m est différent de 1.

2. Composé de formule (I) : 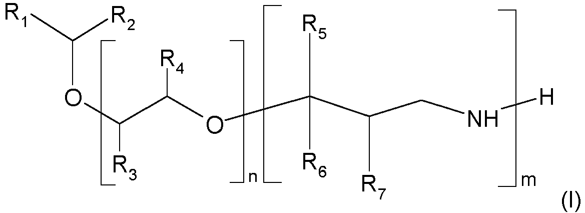 dans laquelle :
- R₁ et R₂, identiques ou différents, représentent, indépendamment l'un de l'autre, un groupement hydrocarboné, linéaire, ramifié ou cyclique, saturé ou insaturé, comprenant de 1 à 15 atomes de carbone, de préférence de 1 à 10 atomes de carbone ;
- R₃ et R₄, identiques ou différents, sont choisis, indépendamment l'un de l'autre, parmi l'atome d'hydrogène, le groupement méthyle et le groupement éthyle ;
- R₅, R₆, et R₇, identiques ou différents, sont choisis, indépendamment l'un de l'autre, parmi l'atome d'hydrogène et un groupement alkyle comprenant de 1 à 6 atomes de carbone, de préférence de 1 à 4 atomes de carbone, de préférence encore de 1 à 3 atomes de carbone ;
- n représente 1 ;
- m est un nombre entier allant de 1 à 6.

3. Composé selon la revendication 2, **caractérisé en ce que** les groupements R₃ et R₄, identiques ou différents, sont choisis, indépendamment l'un de l'autre, parmi l'atome d'hydrogène et le groupement méthyle.

4. Procédé de fabrication d'un composé de formule (I) tel que défini à l'une quelconque des revendications 1 à 3, comprenant successivement :
- une étape de réaction d'un composé de formule (II) : 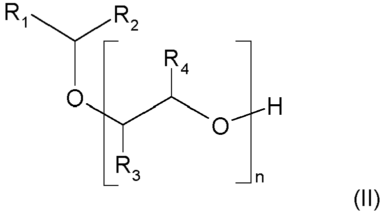 dans laquelle les groupements R₁, R₂, R₃ et R₄ et n sont tels que définis dans la revendication 1 ou la revendication 2 ;
avec un nitrile α,β-insaturé ;
- une réaction d'hydrogénation ;
- le produit issu de ces étapes étant susceptible de réagir en série (m-1) fois avec le nitrile α,β-insaturé puis du dihydrogène, m étant tel que défini dans la revendication 1.

5. Procédé selon la revendication 4, **caractérisé en ce que** le nitrile α,β-insaturé est choisi parmi l'acrylonitrile et le méthacrylonitrile, de préférence l'acrylonitrile.

6. Procédé selon la revendication 4 ou 5, **caractérisé en ce que** le ratio molaire du nitrile α,β-insaturé sur le composé de formule (II) varie de 0,8 à 1,2, de préférence de 0,9 à 1,2.

7. Procédé selon l'une des revendications 4 à 6, **caractérisé en ce que** le ratio molaire du nitrile α,β-insaturé sur le composé de formule (II) varie de 1,01 à 1,1.

8. Procédé selon l'une des revendications 4 à 7, **caractérisé en ce que** la réaction dudit composé de formule (II) avec le nitrile α,β-insaturé est réalisée en présence d'au moins un catalyseur basique (CB).

9. Procédé selon la revendication 8, **caractérisé en ce que** le catalyseur basique (CB) est choisi parmi les hydroxydes d'alcalins et d'alcalino-terreux, les alcoolates alcalins, les hydrures d'alcalins, les résines basiques et les hydroxydes d'ammonium quaternaires, et, de préférence, le catalyseur basique (CB) est choisi parmi l'hydroxyde de sodium, l'hydroxyde de potassium, l'hydrure de sodium et l'hydrure de potassium.

10. Procédé selon l'une quelconque des revendications 4 à 9, **caractérisé en ce que** ledit procédé comprend, préalablement aux étapes successives de réaction du composé de formule (II), tel que défini à la revendication 5, avec un nitrile α,β-insaturé et d'hydrogénation, une étape de réaction d'un alcool de formule (III) :
R₁-CH(OH)-R₂ (III),
où R₁ et R₂ sont tels que définis dans la revendication 1 ou la revendication 2,
avec n composé(s) de formule (IV) : 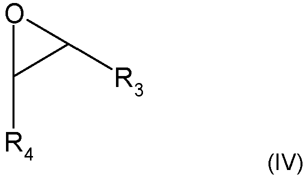
dans laquelle :
- les groupements R₃ et R₄ sont tels que définis dans la revendication 1 ou la revendication 2, et
- n est tel que défini dans la revendication 1 ou la revendication 2.

11. Composé de formule (V) : 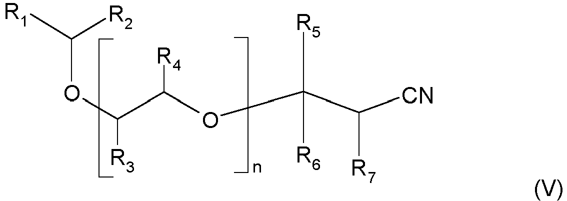 dans laquelle :
- les groupements R₁, R₂, R₃, R₄, R₅, R₆ et R₇ sont tels que définis dans la revendication 1 ou la revendication 2 ;
- n est tel que défini dans la revendication 1 ou la revendication 2.

12. Composé de formule (VI) : 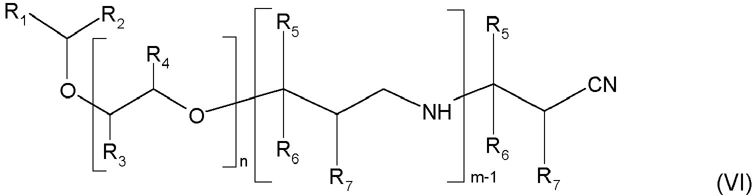 dans laquelle :
- les groupements R₁, R₂, R₃, R₄, R₅, R₆ et R₇ sont tels que définis dans la revendication 1 ou la revendication 2 ;
- n et m sont tels que définis dans la revendication 1 ou la revendication 2.

13. Utilisation d'un composé de formule (I) tel que défini à l'une quelconque des revendications 1 à 3, en tant que lubrifiant, tensioactif cationique, collecteur de flottation pour les minerais, inhibiteur de corrosion, additif pour carburant et agent de réticulation pour les résines époxy.

## Patentansprüche

1. Verbindung der Formel (I): 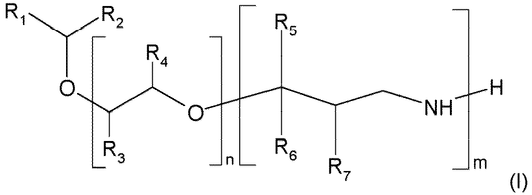 in der:
- R₁ für eine Hexylgruppe steht, R₂ für eine Methylgruppe steht,
- die Gruppen R₃ und R₄ gleich oder verschieden sind und unabhängig voneinander aus einem Wasserstoffatom, einer Methylgruppe und einer Ethylgruppe ausgewählt sind;
- die Gruppen R₅, R₆ und R₇ jeweils für ein Wasserstoffatom stehen;
- n für 0 steht;
- m für eine ganze Zahl im Bereich von 1 bis 6 steht und m von 1 verschieden ist.

2. Verbindung der Formel (I): 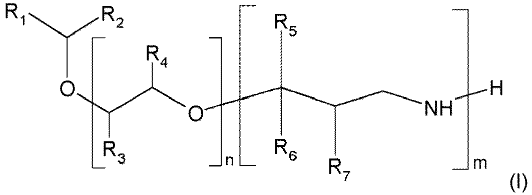 in der:
- R₁ und R₂ gleich oder verschieden sind und unabhängig voneinander für eine lineare, verzweigte oder cyclische, gesättigte oder ungesättigte Kohlenwasserstoffgruppe mit 1 bis 15 Kohlenstoffatomen, vorzugsweise 1 bis 10 Kohlenstoffatomen, stehen;
- R₃ und R₄ gleich oder verschieden sind und unabhängig voneinander aus einem Wasserstoffatom, einer Methylgruppe und einer Ethylgruppe ausgewählt sind;
- R₅, R₆ und R₇ gleich oder verschieden sind und unabhängig voneinander aus einem Wasserstoffatom und einer Alkylgruppe mit 1 bis 6 Kohlenstoffatomen, vorzugsweise 1 bis 4 Kohlenstoffatomen, noch weiter bevorzugt 1 bis 3 Kohlenstoffatomen, ausgewählt sind;
- n für 1 steht;
- m für eine ganze Zahl im Bereich von 1 bis 6 steht.

3. Verbindung nach Anspruch 2, **dadurch gekennzeichnet, dass** die Gruppen R₃ und R₄ gleich oder verschieden sind und unabhängig voneinander aus einem Wasserstoffatom und einer Methylgruppe ausgewählt sind.

4. Verfahren zur Herstellung einer Verbindung der Formel (I) gemäß einem der Ansprüche 1 bis 3, die nacheinander Folgendes umfasst:
- einen Schritt der Umsetzung einer Verbindung der Formel (II): 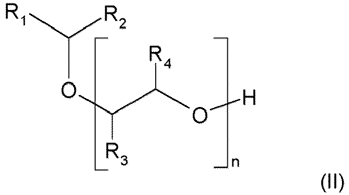 in der die Gruppen R₁, R₂, R₃ und R₄ wie in Anspruch 1 oder Anspruch 2 definiert sind; mit einem α,β-ungesättigten Nitril;
- eine Hydrierungsreaktion;
- wobei das Produkt aus diesen Schritten (m-1)-mal mit dem α,β-ungesättigten Nitril und dann mit Diwasserstoff reagieren kann, wobei m wie in Anspruch 1 definiert ist.

5. Verfahren nach Anspruch 4, **dadurch gekennzeichnet, dass** das α,β-ungesättigte Nitril aus Acrylnitril und Methacrylnitril, vorzugsweise Acrylnitril, ausgewählt wird.

6. Verfahren nach Anspruch 4 oder 5, **dadurch gekennzeichnet, dass** das Molverhältnis von α,β-ungesättigtem Nitril zu Verbindung der Formel (II) im Bereich von 0,8 bis 1,2, vorzugsweise von 0,9 bis 1,2, liegt.

7. Verfahren nach einem der Ansprüche 4 bis 6, **dadurch gekennzeichnet, dass** das Molverhältnis von α,β-ungesättigtem Nitril zu Verbindung der Formel (II) im Bereich von 1,01 bis 1,1 liegt.

8. Verfahren nach einem der Ansprüche 4 bis 7, **dadurch gekennzeichnet**, **dadurch gekennzeichnet, dass** die Umsetzung der Verbindung der Formel (II) mit dem α,β-ungesättigten Nitril in Gegenwart mindestens eines basischen Katalysators (BK) durchgeführt wird.

9. Verfahren nach Anspruch 8, **dadurch gekennzeichnet, dass** der basische Katalysator (BK) aus Alkali- und Erdalkalihydroxiden, Alkalialkoholaten, Alkalihydriden, basischen Harzen und quartären Ammoniumhydroxiden ausgewählt wird und der basische Katalysator (BK) vorzugsweise aus Natriumhydroxid, Kaliumhydroxid, Natriumhydrid und Kaliumhydrid ausgewählt wird.

10. Verfahren nach einem der Ansprüche 4 bis 9, **dadurch gekennzeichnet, dass** das Verfahren vor den aufeinanderfolgenden Schritten der Umsetzung der Verbindung der Formel (II) gemäß Anspruch 5 mit einem α,β-ungesättigten Nitril und Hydrierung einen Schritt der Umsetzung eines Alkohols der Formel (III) :
R₁-CH(OH)-R₂ (II),
wobei R₁ und R₂ wie in Anspruch 1 oder Anspruch 2 definiert sind,
mit n Verbindung(en) der Formel (IV): 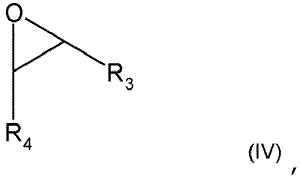
in der:
- die Gruppen R₃ und R₄ wie in Anspruch 1 oder Anspruch 2 definiert sind und
- n wie in Anspruch 1 oder Anspruch 2 definiert ist,
umfasst.

11. Verbindung der Formel (V): 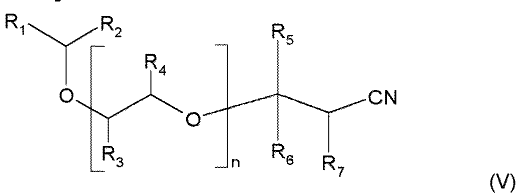 in der:
- die Gruppen R₁, R₂, R₃, R₄, R₅, R₆ und R₇ wie in Anspruch 1 oder Anspruch 2 definiert sind;
- n wie in Anspruch 1 oder Anspruch 2 definiert ist.

12. Verbindung der Formel (VI): 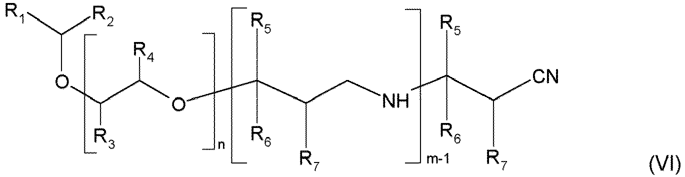 in der:
- die Gruppen R₁, R₂, R₃, R₄, R₅, R₆ und R₇ wie in Anspruch 1 oder Anspruch 2 definiert sind;
- n und m wie in Anspruch 1 oder Anspruch 2 definiert sind.

13. Verwendung einer Verbindung der Formel (I) gemäß einem der Ansprüche 1 bis 3 als Schmiermittel, kationisches Tensid, Flotationssammler für Mineralien, Korrosionsinhibitor, Kraftstoffadditiv und Vernetzungsmittel für Epoxidharze.

## Claims

1. Compound of formula (I): 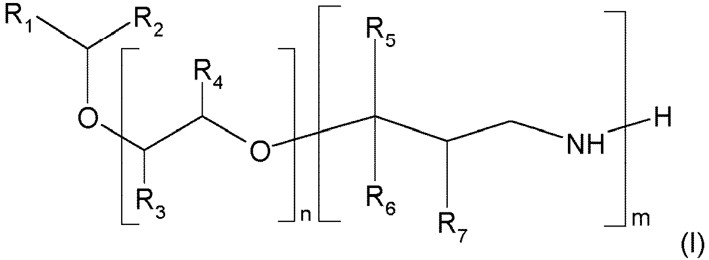 in which:
- R₁ is a hexyl group, R₂ is a methyl group;
- the groups R₃ and R₄, which may be identical or different, are chosen independently of one another from a hydrogen atom, a methyl group and an ethyl group;
- the groups R₅, R₆ and R₇ each represent a hydrogen atom;
- n is 0;
- m is an integer ranging from 1 to 6, and m is other than 1.

2. Compound of formula (I): 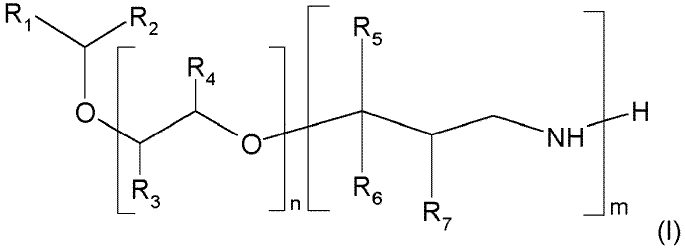 in which:
- R₁ and R₂, which may be identical or different, independently of one another represent a linear, branched or cyclic, saturated or unsaturated hydrocarbon-based group, comprising from 1 to 15 carbon atoms, preferably from 1 to 10 carbon atoms;
- R₃ and R₄, which may be identical or different, are chosen independently of one another from a hydrogen atom, a methyl group and an ethyl group;
- R₅, R₆ and R₇, which may be identical or different, are chosen independently of one another from a hydrogen atom and an alkyl group comprising from 1 to 6 carbon atoms, preferably from 1 to 4 carbon atoms, more preferably from 1 to 3 carbon atoms;
- n represents 1;
- m is an integer ranging from 1 to 6.

3. Compound according to Claim 2, **characterized in that** the groups R₃ and R₄, which may be identical or different, are chosen independently of one another from a hydrogen atom and a methyl group.

4. Process for producing a compound of formula (I) as defined in any one of Claims 1 to 3, comprising, successively:
- a step of reaction of a compound of formula (II): 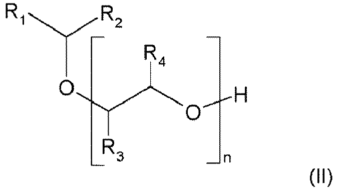
in which the groups R₁, R₂, R₃ and R₄ and n are as defined in Claim 1 or Claim 2;
with an α,β-unsaturated nitrile;
- a hydrogenation reaction;
- the product obtained from these steps being capable of reacting in series (m-1) times with the α,β-unsaturated nitrile and then with dihydrogen, m being as defined in Claim 1.

5. Process according to Claim 4, **characterized in that** the α,β-unsaturated nitrile is chosen from acrylonitrile and methacrylonitrile, preferably acrylonitrile.

6. Process according to Claim 4 or 5, **characterized in that** the molar ratio of the α,β-unsaturated nitrile to the compound of formula (II) ranges from 0.8 to 1.2, preferably from 0.9 to 1.2.

7. Process according to one of Claims 4 to 6, **characterized in that** the molar ratio of the α,β-unsaturated nitrile to the compound of formula (II) ranges from 1.01 to 1.1.

8. Process according to one of Claims 4 to 7, **characterized in that** the reaction of said compound of formula (II) with the α,β-unsaturated nitrile is carried out in the presence of at least one basic catalyst (CB).

9. Process according to Claim 8, **characterized in that** the basic catalyst (CB) is chosen from alkali metal and alkaline earth metal hydroxides, alkali metal alkoxides, alkali metal hydrides, basic resins and quaternary ammonium hydroxides, and, preferably, the basic catalyst (CB) is chosen from sodium hydroxide, potassium hydroxide, sodium hydride and potassium hydride.

10. Process according to any one of Claims 4 to 9, **characterized in that** said process comprises, prior to the successive steps of reaction of the compound of formula (II), as defined in Claim 5, with an α,β-unsaturated nitrile and hydrogenation, a step of reaction of an alcohol of formula (III):
R₁-CH(OH)-R₂ (III),
where R₁ and R₂ are as defined in Claim 1 or Claim 2, with n compound(s) of formula (IV): 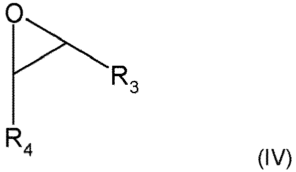 in which:
- the groups R₃ and R₄ are as defined in Claim 1 or Claim 2, and
- n is as defined in Claim 1 or Claim 2.

11. Compound of formula (V): 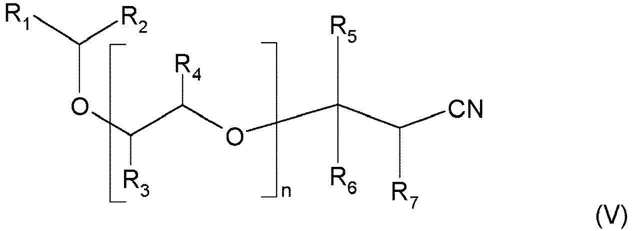 in which:
- the groups R₁, R₂, R₃, R₄, R₅, R₆ and R₇ are as defined in Claim 1 or Claim 2;
- n is as defined in Claim 1 or Claim 2.

12. Compound of formula (VI): 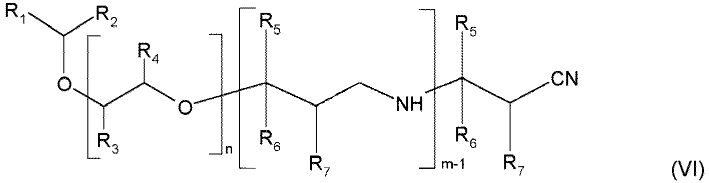 in which:
- the groups R₁, R₂, R₃, R₄, R₅, R₆ and R₇ are as defined in Claim 1 or Claim 2;
- n and m are as defined in Claim 1 or Claim 2.

13. Use of a compound of formula (I) as defined in any one of Claims 1 to 3 as lubricant, cationic surfactant, ore flotation collector, corrosion inhibitor, fuel additive and crosslinking agent for epoxy resins.
